# EUROPEAN PATENT SPECIFICATION

(11) **EP 1 830 719 B1**
(45) Date of publication and mention of the grant of the patent: **08.07.2009**
(21) Application number: 05854449.5
(22) Date of filing: 15.12.2005
(51) Int. Cl.: A61B 17/22

(54) **RADIOPAQUE MANIPULATION DEVICES**
RÖNTGENDICHTE MANIPULATIONSVORRICHTUNGEN
DISPOSITIFS DE MANIPULATION RADIO-OPAQUES

(30) Priority: 15.12.2004 US 636411 P
(43) Date of publication of application: 12.09.2007
(73) Proprietor: Cook Urological Inc., Spencer IN 47460 (US); Cook Ireland Ltd, Limerick (IE)
(72) Inventor: LAVELLE, Shay, Annacotty, County Lemerick (IE)
(74) Representative: Garratt, Peter Douglas
(86) International application number: PCT/US2005/045733
(87) International publication number: WO 2006/066114

(56) References cited:
- US-A- 6 066 149
- US-A1- 2002 123 765
- US-A1- 2004 122 445

## Description

### TECHNICAL FIELD

The technical field of the invention is that of minimally-invasive medical devices.

### BACKGROUND

There is a continuing need for instruments to diagnose and treat people by means of minimally-invasive surgical procedures. For example, various organs and passages in the body are subject to the development of stones, calculi and the like. Kidney stones are a common problem in the United States. Kidney stones are painful and are the most frequent cause of kidney inflammation. Calculi and concretions in other parts of the biliary system are also commonplace. Similarly, stones, calculi, concretions and the like can develop throughout the renal or urinary system, not only in the ureters and distal to them, but also in the renal tubules and in the major and minor renal calyxes.

Minimally invasive surgical procedures have been developed for the removal of stones, calculi, concretions and the like from the biliary, vascular, and urinary systems, as well as for the removal or retrieval of foreign bodies from a variety of locations in the body. Such procedures avoid the performance of open surgical procedures such as, for example, an anatrophic nephrolithotomy.

Minimally invasive procedures can instead employ percutaneous access, in which stones, calculi, concretions, foreign bodies and the like are removed through a percutaneously inserted access sheath. Several access routes are suitable, depending upon the specific system and the particular location in the system at which the stones, calculi, concretions, foreign bodies or the like are found. One access route that is infrequently used is direct percutaneous insertion of a retrieval device to remove calculi and kidney stones.

Without regard to the particular access route, percutaneous extraction may be based upon the use of catheters or similar devices to engage and remove the stones, calculi, concretions, foreign bodies and the like. Such catheters and devices typically comprise a hollow, flexible sheath and a retrieval device at the distal end of an inner cannula. The retrieval device may be a basket comprising a plurality of wires positioned in and extendable from the sheath. The wires are joined or arranged so as to form a basket or forceps for engaging the object to be retrieved when the wires are extended from the sheath. The basket can be collapsed by withdrawing the wires into the sheath. A helical basket permits entry of the stone or the like from the side of the basket, while an open ended ("eggwhip") basket allows a head-on approach to the stone or the like. Reference is hereby directed to US 6 066 149 which describes a surgical device which may be used to open a clear passageway adjacent thrombus to allow both blood and medication to bypass the clot or to pierce and to remove thrombus. The device has a core element, typically a core wire. Placed around the distal end of the core element is a collapsible but preferably self expanding proximal cage assembly and a distal filter, preferably a self-expanding cage assembly or other filter component. The various portions of those components are preferably radio-opaque. Further reference is directed to US 2002/123765 in which devices and methods for removing an obstruction from a blood vessel are described. The devices are deployed in a collapsed condition and are then expanded within the body. The devices are then manipulated to engage and remove the obstruction.
Further reference is directed to US 2004/122445 in which a rigid extractor is revealed, a rigid device for use in percutaneous procedures to remove kidney stones directly from the kidneys. The rigid extractor uses an outer rigid cannula and an inner cannula to control a basket retriever for removing kidney stones and calculi from a kidney of a patient. The extractor is desirably used with a fluoroscope, in which the surgeon maneuvers the extractor with the aid of a view of the operating field provided by the fluoroscope.

Other retrievers and graspers can include forceps or can include a loop or snare for encircling the body to be removed, the loop or snare being made of the wire. Such devices may be used in conjunction with a nephroscope, to aid the physician in seeing the operating field. Using such a device also tends to limit the size of the cannula and basket used.

Despite their successful use for some time, such retrieval devices are subject to drawbacks. The principal device that is used to retrieve kidney stones is a 3-pronged grasper. The prongs of the grasper, useful in grasping stones, may cause damage to kidney or contiguous tissue, leading to bleeding, and potentially significantly extending the time for the procedure. The very flexible, movable nature of these graspers adds to the problem, in that their flexibility and mobility make them more difficult to control. One particular aspect that makes these devices difficult to control is the fact that these devices are typically made of stainless steel, or of superelastic shape memory alloys, such as Nitinol-type alloys or shape memory polymers. Instruments made from these alloys are poorly visible under x-ray or fluoroscopy, and surgeons are not able to trace the position of the instrument, or the end-effector as well as they might wish. If the instrument is being used with an endoscope or similar device, the field of view may, in particular situations, be highly restricted, and subsequent visibility under fluoroscopy and x-rays becomes necessary.

It would be highly desirable to have a device suitable for manipulating tissue or other objects inside the human body that is easier to observe for the capture and retrieval or extraction of kidney stones, or for a variety of other medical procedures. The device would ideally also be safe and effective.

### BRIEF SUMMARY

One aspect of the invention is a retrieval or manipulation device. The device includes a control rod and a retrieval device attached to the control rod, the retrieval device including a plurality of retrieving elements made from a plurality of wires, at least one wire in the retrieval device made from a radiopaque material. The device includes a sheath and is configured so that when the sheath is retracted or the retrieval device is extended, the retrieval device extends from the sheath. The at least one radiopaque wire includes a superelastic radiopaque alloy with from about 3 to about 14 percent opacifying element, and either about 50 percent titanium and the balance nickel, or with about 50 percent nickel and the balance titanium.

Another aspect of the invention is a medical manipulation device. The medical manipulation device includes a control rod, a plurality of superelastic wire loops attached to the control rod, the wire loops formed into a basket with an atraumatic periphery, and with at least one of the wire loops comprising a radiopaque wire. The device also includes a sheath and is configured so that when the sheath is retracted or the basket is extended, the basket expands, and the loops are in a relaxed condition when outside the sheath.

Another aspect of the invention is a method of making a radiopaque retrieval device. The method includes forming a plurality of retrieving elements, the retrieving elements made from a plurality of wires, at least one wire in the retrieving elements made from a radiopaque material. The method also includes assembling the retrieving elements into a retrieval device, and attaching the retrieval device to a control rod. There are many aspects of the invention, a few of which are described in the drawings and explanations below.

### BRIEF DESCRIPTION OF THE DRAWINGS

Fig. 1 is a stress-strain curve for a superelastic alloy, showing the characteristic flag shape;

Fig. 2 depicts a stress-strain curve for a convention metal alloy;

Figs. 3a-3b depict the use of radiopaque wires in a wire bundle with non-radiopaque wires;

Figs. 3c-3d depict the use of radiopaque plating on a non-radiopaque wire;

Figs. 4a-4f depict several additional embodiments of baskets and retrieval devices made from superelastic radiopaque wire;

Fig. 5 depicts a basket retrieving kidney stones;

Fig. 6 depicts a manipulation device useful for retrieving a stone from a common bile duct;

Figs. 7a-7d depict alternate configurations of manipulation devices;

Fig. 8 depicts a ureteral backstop filter and retrieval device, the device made easier to track inside a body because of its radiopacity;

Fig. 9 depicts another embodiment with a 4-wire basket made from radiopaque nitinol;

Fig. 10 depicts a spiral trap made from a radiopaque nitinol wire;

Figs. 11a -11c depict a three-prong grasper made from at least one radiopaque wire;

Figs. 12a-12b depict a four-prong grasper made from at least one radiopaque wire;

Figs. 13a-13e depict a process for making a radiopaque manipulation device by a metal-removal process; and

Figs. 14a-14c depict an alternative process for making a radiopaque manipulation device by a metal-removal process.

### DETAILED DESCRIPTION OF THE DRAWINGS AND THE PRESENTLY PREFERRED EMBODIMENTS

There are many embodiments of the present invention, of which the drawings and this description present only a few. Embodiments of manipulation or retrieval devices may include wires that are made with a highly radiopaque core material, devices that include radiopaque markers or portions that are radiopaque, and retrieval devices that are plated with a radiopaque material. Because these devices are expensive or prone to other problems, retrieval devices made from radiopaque wires are preferred. A radiopaque component is defined as a component that is more visible in x-ray or fluoroscopic images than a comparable component made from a 50/50 atomic percentage alloy of nickel and titanium. It is also noted that standard Nitinol alloys may include a little less titanium, a slightly higher nickel content (up to about 50.25%) and a small amount of chromium (up to about 0.25%) for improved superelasticity properties. These small variations have no noticeable effect on radiopacity, and the small amount of chromium is not meant as the "third" metal in an alloy for imparting radiopacity.

Radiopaque wires may be made by alloying nickel and titanium with another metallic element (metal) or a combination of metallic elements (metals). The preferred alloys may include a 49.8 to 51.5 atomic percent nickel, a small percentage of tungsten, tantalum, palladium, platinum, gold, iridium, rhenium, rhodium, silver, osmium, ruthenium, copper, iron, vanadium, chromium, zirconium, niobium, molybdenum, and hafnium, such as 0.5 to 2%, and the remainder titanium. Other suitable alloys include those with 49.0 to 51.0 atomic percent nickel, 2 to 20 atomic percent tungsten, tantalum, palladium, platinum, gold, iridium, rhenium, rhodium, silver, osmium, ruthenium, vanadium, copper, iron, chromium, zirconium, niobium, molybdenum, and hafnium, and the balance titanium. Additional suitable alloys include those with 34.0 to 49.0 atomic percent nickel, about 3 to 14 percent Pd, and the balance titanium. In general, heavier metals, such as tungsten, tend to be more radiopaque than lighter metals, such as titanium. Particularly preferred alloying elements are the less-expensive metals, such as tungsten and tantalum, rather than metals that are effective at imparting radiopacity but are more expensive, such as palladium, platinum or gold.

Other radiopaque alloys may include 34-49 percent Ni, 3-14 percent of an opacifying element, and the balance Ti. The preferred opacifying elements may include Ir, Rh, Pt, Cu, Au, Ag, Fe, Os, and Ru. Other alloys may include about 49-51 percent Ni, about 2-20 percent Ir, Rh, Pt, Pd, Cu, Au, Ag, Fe, Os and Ru, and the balance Ti. Still other alloys may include 49.8 to 51.5 atomic percent Ni, a small amount, about 0.5 to 2% of opacifying element, and the balance Ti. Opacifying elements for all these alloys preferably include Ir, Rh, Pt, Pd, Cu, Au, Ag, Fe, Os, and Ru. Other opacifying elements for these alloys may include Ta, W, Nb, Zr, V, Cr, Mo, Hf and Re.

Other radiopaque alloys may include 49-51 percent Ni, about 3-14 percent opacifying element, and the balance Ti. The preferred opacifying elements are preferably Ta, W, Nb, Zr, V, Cr, Mo, Hf and Re. Additional radiopaque alloys may include Ir, Rh, Pt, Pd, Cu, Au, Ag, Fe, Os and Ru. Still other radiopaque alloys may include about 49-51 percent Ni, about 2- 20 percent of an opacifying element, and the balance Ti. The opacifying element is preferably one of Ta, W, Nb, Zr, V, Cr, Mo, Hf and Re.

In addition, finished wire, or a finished product made from wire, may be plated with a thin coating of a metal that is radiopaque. While many metals are possible, plating solutions are readily available for Au, Ag, Cu, Pd, Pt, Rh and Re, among others. Thus, radiopaque graspers and retrieval devices may include those devices made radiopaque by plating a radiopaque coating onto superelastic wire or onto a device made from superelastic wire.

In order to keep the size of the basket and the diameter of the sheath narrow, very thin wires are preferred, preferably wires having a diameter of about 0.0025 inches (about 0.063 mm) or less, but wires of any diameter may be used. Round wires are preferred, but wires of any shape may be used, including rectangular wire, square wire, wedge or "pie-shaped" wire, flat wire and triangular wire. Each "wire" depicted in the retrieval device embodiments disclosed herein may comprise two or more wires twisted together for greater stiffness and control of the device. In other embodiments, a flat wire, for instance, may be used for one arm of a grasper.

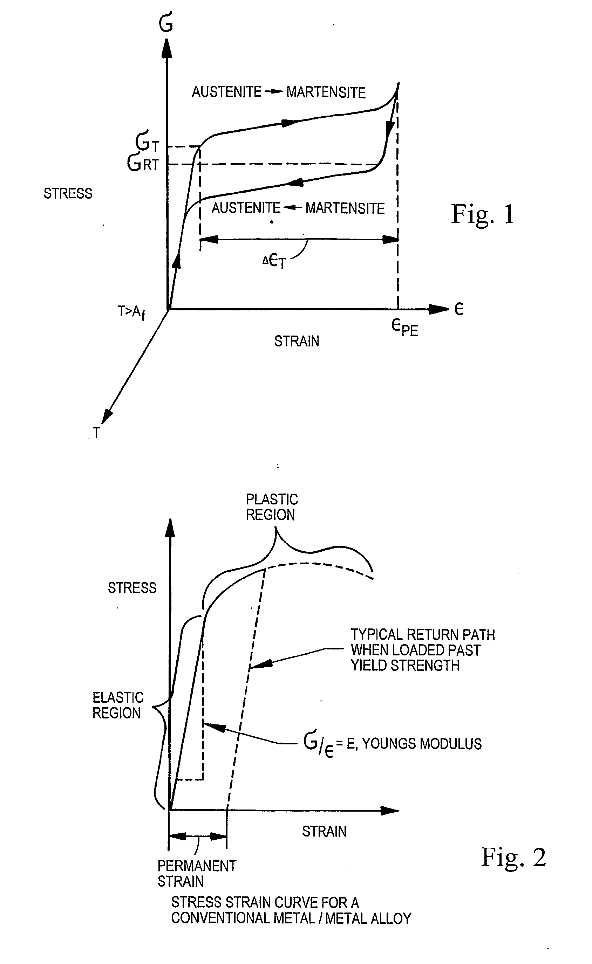

Metallic superelastic alloys have a characteristic "flag" shape in their stress-strain diagrams, as shown in Fig. 1, in the elastic deformation region, i.e., the region of lower stress and strain. Pseudoelasticity is generally defined as nonlinearity or hysteresis between the upper or loading curve, and the lower or unloading curve. As shown in Fig. 1, the upper stress-strain curve generally comprises an "upper plateau" in which stress is relatively constant while strain increases. The lower stress-strain curve comprises a lower plateau, at a relatively constant, lower rate of stress, while strain decreases. Non-pseudoelastic metals or alloys tend to have some minimal hysteresis in their loading and unloading stress strain curves, but not a plateau, in the elastic deformation region.

Figs. 2a and 2b additionally depict graphically the mechanical behavior of superelastic alloys. Fig. 2a compares generally the behavior of superelastic alloys compared to stronger (austenitic) and weaker (martensitic) alloys. Fig. 2b depicts a stress-strain curve for a conventional metal, showing a lower-stress/strain elastic region, and a region of higher stress and strain, the plastic region. A specimen or a part may be exercised in the elastic region, and it will return to its beginning shape, i.e., it will "elastically deform." Once the specimen or part has been exercised into the "plastic" region, it may be permanently deformed, and will not automatically return to its former shape. In the same manner, a superelastic alloy will behave elastically if its deformation or exercise is confined to the elastic region. It has been found in Ni-Ti-Pd alloys, with about 40-43 atomic percent nickel, 50 atomic percent titanium, and about 7 to 10 percent palladium, that the A_{f} temperature is from about -20 to +20°C. This is the temperature at which the reverse Martensite transformation is complete, and is desirable for achieving the superelastic alloy effect when a medical device is placed into a patient for a medical procedure.

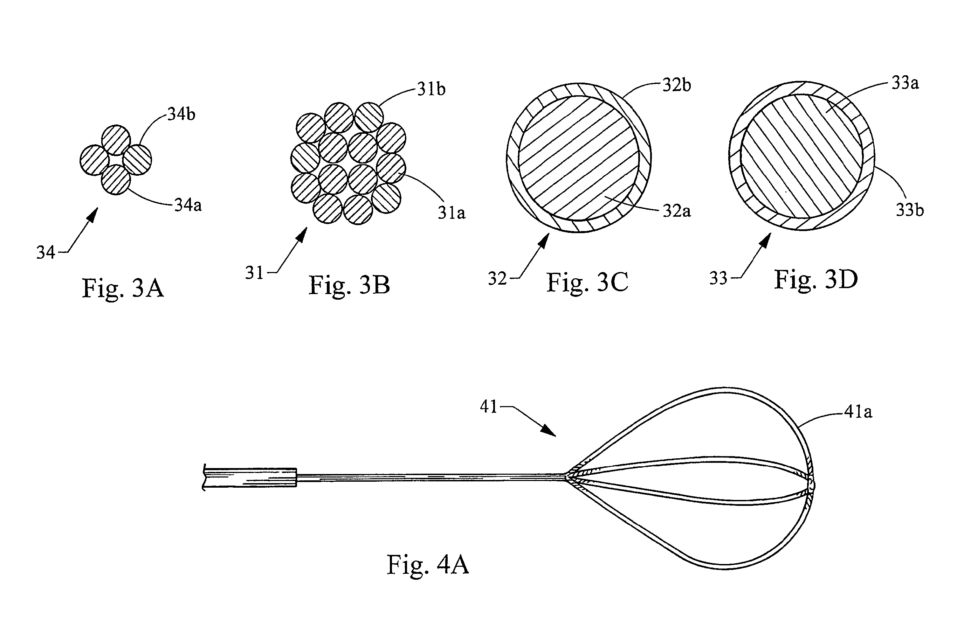

Examples of urinary tract stone manipulation devices taking advantage of the present invention are shown in Figs. 3a-3d. These include tipped and virtually tipless baskets using various cross sectional shapes of wires (e.g. circular, pie- shaped (Delta®, flat etc.). The formed configuration of wires includes circular arch, helical, knitted bundles, interwoven, and so forth. Typical basket devices generally consist of a number of wires at the device distal extremity, which encapsulate the space intended to entrap a target object. The wires may connect together and terminate at the distal extremity of the basket portion of the device. On the proximal end, the wires join may through a coupling to a control rod, which extends to a handle of the device. The control rod is preferably placed within a sheath. When the sheath is advanced or withdrawn, the basket or the sheath extends or retracts. Various types of operating handles can be connected at the proximal end of the device to aid the co-axial movement of the control rod within the sheath.

Other urinary tract stone manipulation devices include graspers, which are open ended baskets, and entrapment devices, which are used to minimize stone migration during lithotripsy procedures. In lithotripsy, a stone is fragmented, often by a laser, while using an entrapment device to prevent retrograde drift of the broken particles into the kidney. Subsequent removal of multiple fragments can be a tedious task requiring multiple endoscope passes and associated with patient discomfort.

Figs. 3a and 3b also depict wire bundles and cross sections of wires useful in embodiments of the present invention. In Figs. 3a and 3b, wire bundle 31 includes several non-radiopaque wires 31 a which have been assembled or grouped with radiopaque wire 31 b to render wire bundle 31 radiopaque. Wire bundle 34 includes three non-radiopaque wires 34a and a single radiopaque wire 34b. The single radiopaque wire 34b enables medical personnel to more readily see the wire bundle when using fluoroscopy or x-ray techniques. As shown in Figs. 3c and 3d, a non-radiopaque wire may be rendered radiopaque by using a radiopaque coating or by using radiopaque material to form the wire. Wires 32 and 33 are radiopaque because they include a radiopaque coating 32b, 33b over a non-radiopaque Nitinol core 32a, 33a.

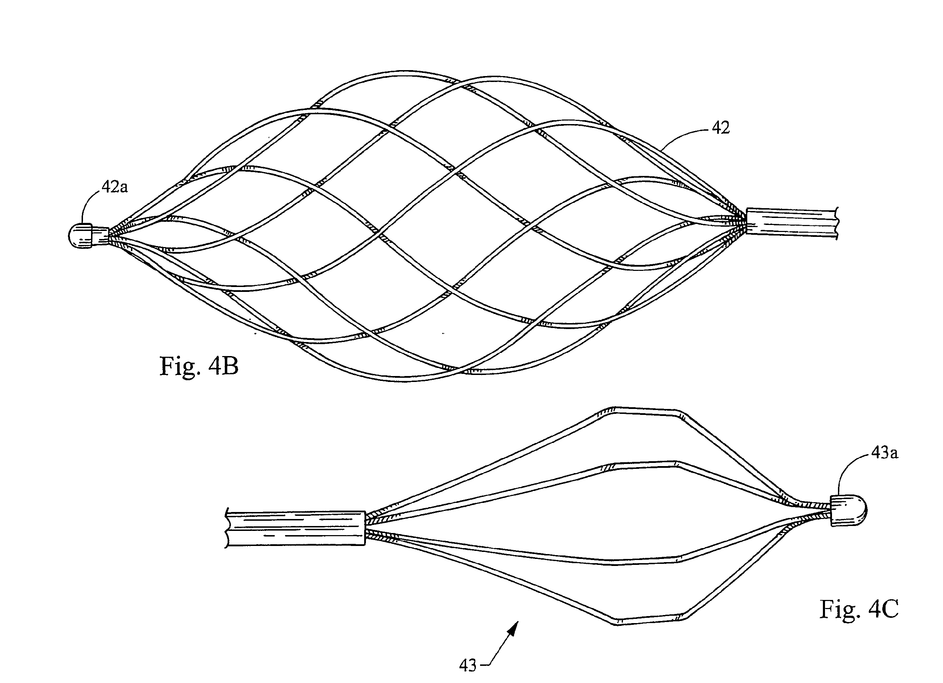

Examples of biliary duct stone manipulation devices are shown in Figs. 4a-4e. Similar to their urinary tract counterparts, the wire profiles and formed configurations of the biliary duct devices have numerous configurations. The devices also operate in a similar manner to the urinary tract products. Fig. 4a depicts a direct access system (minimally-invasive) extraction basket 41, made from wires 41 a with radiopaque Nitinol alloy or coated with a radiopaque plating or cladding. Atraumatic baskets that have very small tips are described in copending U.S. Pat. Appl. 10/679,007, (US 2004/0122445) filed October 3, 2003, the contents of which are incorporated herein by reference. A "Memory Basket" 42 made from eight wires with radiopaque Nitinol alloy or coated with a radiopaque plating or cladding is depicted in Fig. 4b. The basket may have a slight filiform 42a as shown, or may be virtually tipless, as is the basket of Fig. 4a.

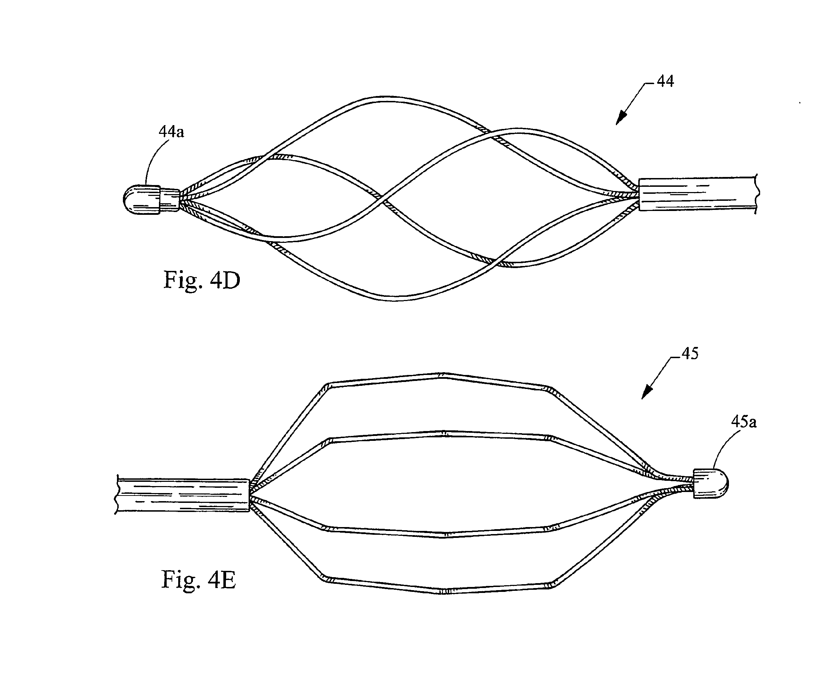

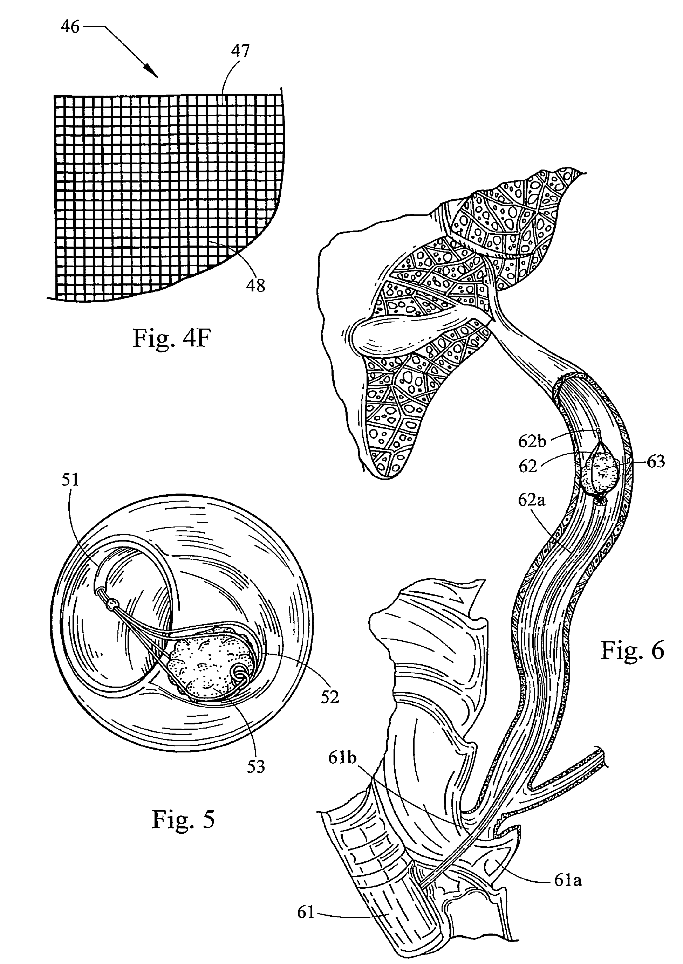

Another embodiment of a basket 43 with 5 Fr radiopaque wire is depicted in Fig. 4c. The basket has a slight filiform 43a, and is made from multi-stranded wire (a "soft wire" configuration), rather than from a single larger filament. A spiral-wound basket 44 with a slight filiform 44a is depicted in Fig. 4d. This basket is made from 7 Fr radiopaque wire, and is referred to as "hard wire," because the basket made from a single wire has higher stiffness and greater resistance to radial deformation. An extraction basket 45 with four radiopaque wires having a different, more prominent shape is shown in Fig. 4e. This basket also has a filiform 45 a at the distal end. A filter mesh, as shown in Fig. 4f, may also be attached to a retrieval device in some embodiments. Filter mesh 46 may include fine wires 47, 48, woven as shown, in or another desired pattern. At least one wire in each direction is preferably radiopaque.

During the surgical stone manipulation procedure the devices are often endoscopically visible where the target object is in constant view throughout the operation. In some instances the stone manipulation device can be deployed beyond a stone and hence vision is impeded. Figure 5 shows a stone 53 being captured in the kidney where a flexible ureteroscope 51 permits constant direct vision of atraumatic basket 52.

Fig. 6 shows a stone 63 being captured in the bile duct. In this case, a duodenoscope 61 in a nearby intestine 61a permits direct vision of only the papilla 61b through which the sheath 62a, retrieval basket 62 and optional filiform 62b are introduced into the bile duct. Once in the bile duct, the basket is no longer endoscopically visible to the surgeon. A radiopaque basket, which can be observed under fluoroscopy, is very helpful in guiding and manipulating the basket. It should be noted that retrieval and manipulating embodiments may be used to retrieve calculi or stones, and remove them from the body directly. Alternatively, these devices may deal with gallstones, or calculi that form in the bile duct, by crushing or fragmenting them. These calculi may be soft rather than harder kidneystones, and retrieval and manipulating embodiments may be capable of crushing or slicing the gallstones into very small and harmless fragments. Embodiments may thus be used to retrieve and remove calculi or stones, or may be used to move or manipulate them inside the body without directly removing them from the body.

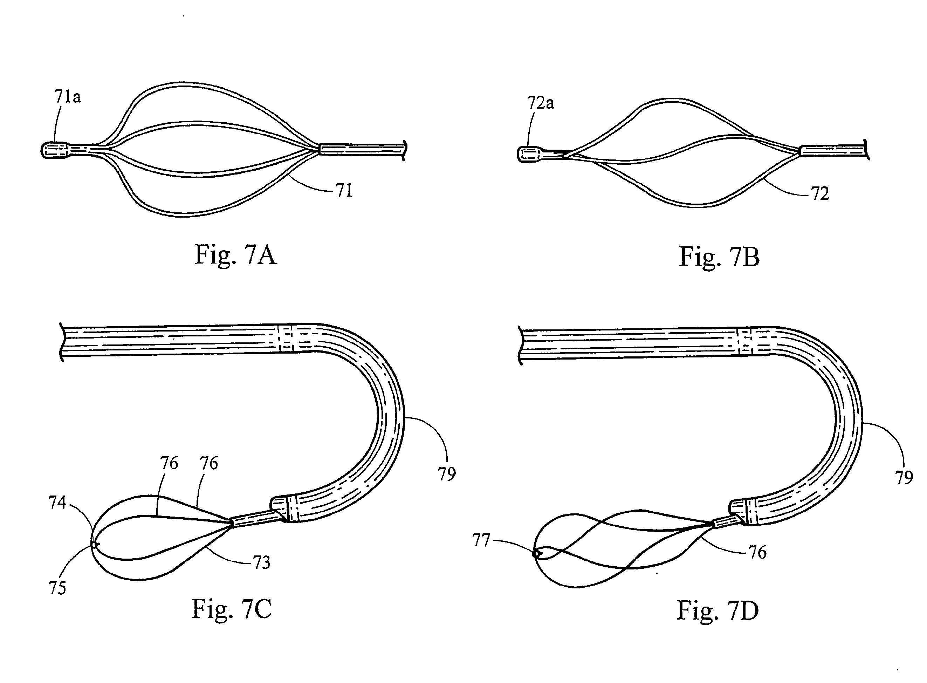

Figs. 7a-7d depict additional embodiments of baskets or retrieval devices in which radiopaque wires are very helpful. Fig. 7a depicts a four-wire basket 71 with a distal filiform 71a. Filiform 71a may be useful in guiding basket 71 as far as a ureter or other body passage. Stones typically are maneuvered to enter the basket using the larger openings near the center of the basket, and are then retained by the smaller opening near the distal end of the basket. Fig. 7b depicts a three-wire spiral basket 72. This embodiment also has larger opening near the center of the basket, and smaller openings near the distal end for retaining stones or other captured matter. The filiform 72a at the distal tip can help guide the basket into a desired body passage.

Figs. 7c and 7d depict atraumatic baskets without a filiform, the baskets being deployed from flexible endoscopes 79. Fig. 7c depicts a four-wire or two-loop basket 73. The tip 74 is made atraumatic, the wires being joined by forming one small loop 77 in one of the larger loops 76, the smaller loop made around the other large loop. In Fig. 7c, the wires are relatively straight, while in Fig. 7d, the basket 76 is made from two large spiral shaped loops. In this embodiment, a small loop is formed in one of the large loops, the smaller loop encompassing the other large loop to form a basket. The baskets in Figs. 7c and 7d are shown emerging from the working channel of a very flexible endoscope 79, preferably able to bend upon itself in a 180° angle.

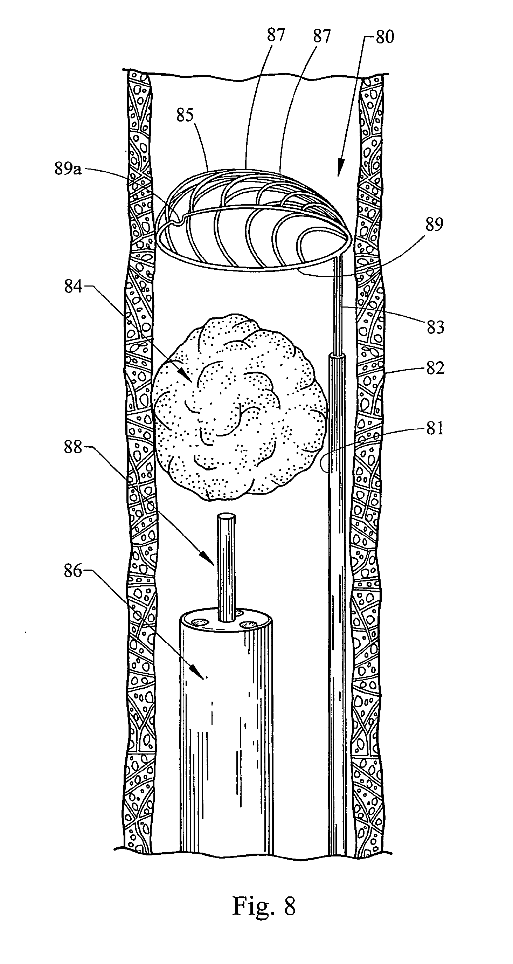

Fig. 8 depicts a ureteral backstop filter and retrieval device. This device is designed to bypass a blockage in a body passage, such as a kidney stone in a ureter. The backstop may be used as part of a laser lithotripsy procedure to fragment the stone and capture the fragments with "backstop filter" aspects of the device. When the basket deploys from its sheath, it expands to one side only, preferably interfacing with the body passage or ureter on all sides to prevent fragments from escaping. Retrieval devices such as this one are described in copending U.S. Pat. Appl. 10/902,754 (US 2005/0043756), filed July 28, 2004, the contents of which are incorporated herein by reference.

A typical use is depicted in Fig. 8. A retrieval device 80 according to the present invention is used in a ureter 82 to trap fragments of a kidney stone 84 when they are broken by an endoscope 86 using a holmium laser 88 or other device. Retrieval device 80 is carried in a sheath 81 and controlled by a control rod 83. There is a basket 85 formed from a plurality of loops 87 at the distal end of retrieval device 80. Loops 87 are preferably interlaced or interleaved among each other to form basket 85. The loops may be interlaced or interleaved by simply going over and under each other in a pattern in which the loops or wires will be trained, or they may also be interlaced by means of smaller loops formed in the larger loops, as will be explained below. Periphery 89 abuts the wall of the ureter or other body vessel and forms a seal to prevent bypassing of objects which should be captured by the retrieval device. Periphery 89 also includes a flex point 89a so that basket 85 can easily fold and collapse into sheath 81.

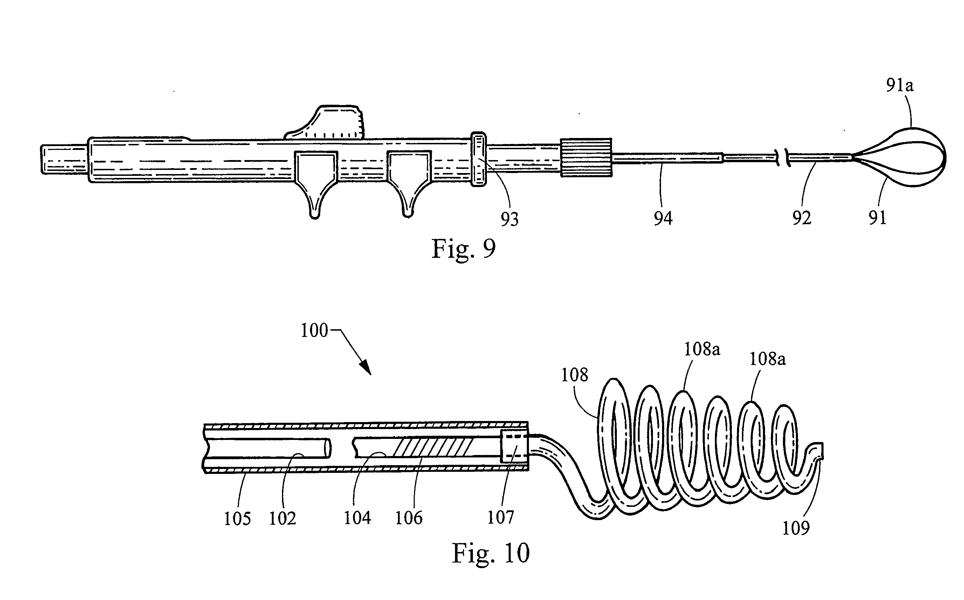

Fig. 9 depicts a four- wire radiopaque Nitinol basket 91 with four loops 91 a, control rod 92, a deployment sheath 94 and a control handle 93. This configuration is preferably used by a surgeon when performing an endoscopic or other minimally-invasive procedure. Fig. 10 depicts a spiral basket, in which the Nitinol wire or wires are shaped into a cone or spiral for capturing stones or other parts to be removed from the body. Retrieval devices such as these are described in copending U.S. Pat. Appl. 10/617,580 (US 2004/0054377), filed March 18, 2004, the contents of which are incorporated herein by reference.

In Fig. 10, a flexible spiral catcher/extractor 100 is made from a control rod or flexible cannula 102 having a distal portion 104 with a helical cut portion 106 and a spiral catcher/extractor 108 with coils 108a at the distal end 109. As described above, there may be a transition portion 107 between the portion having helical cuts 106 and the catcher/extractor 108. In other embodiments, the catcher/extractor may have spiral cuts in the transition portion 107 or in the catcher/extractor portion 108, or in both the transition portion 107 and the catcher/extractor portion 108. The catcher/extractor may be attached or welded to the cannula, or may be integral with the cannula, thus allowing for a more reliable structure and easier manufacture. The cannula and spiral catcher/extractor may form a single continuum of metal and are desirably made from radiopaque nitinol or other super-elastic alloy. The cannula may be used with a separate sheath 105.

The retrieval devices or baskets described above are formed by shaping the wires and loops into the desired shape at room temperature or below, preferably with a cold mandrel, and then annealing the properly-shaped basket at the proper annealing temperature for a time sufficient for the transformation to a superelastic state. In one example, a basket is formed from 0.11 mm diameter (about 0.0043 inches) Ni-Ti-Cu Nitinol wire and is annealed at 990°F (about 530°C) for about 10 minutes. The time and temperature for annealing will vary with the alloy selected and with the diameter (thickness) of the wire. The loops themselves, not merely the annealing oven, must remain at the desired temperature for the proper length of time for the annealing or heat-treatment to be complete. Proper annealing is very important for the wires and the loops to remain kink-free during deployment and operation of the basket. If kinks form for any reason, it may be difficult to deploy (expand) or retract the basket.. It is understood that the retrieval devices are "trained" to assume a relaxed state in the shapes depicted in Figs. 4a-4e, and 5-10. Before deployment from a sheath or other restraint, they may be in a state of stress, seeking to relieve the stress by assuming a relaxed state.

The device is desirably formed before the annealing operation, as discussed above, including all wires or loops desired in the retrieval device. If the basket or retrieval device has a non-symmetrical shape, such as the shape depicted in Fig. 8 above, it is possible that it may require more force or more built-in stress in the wires to reliably emerge from the sheath in the desired shape. Therefore, the annealing or heat-treating operation is even more important than normal in building stresses into the wires and the basket. The alloys preferably have a transition temperature below room temperature, such as about 32°F (0°C), so that the baskets are always in the austenite stage at room temperature and at body temperature. The baskets tend to be confined when placed into the body and are allowed to expand by withdrawing the sheath or extending the basket or other retrieval device from the sheath.

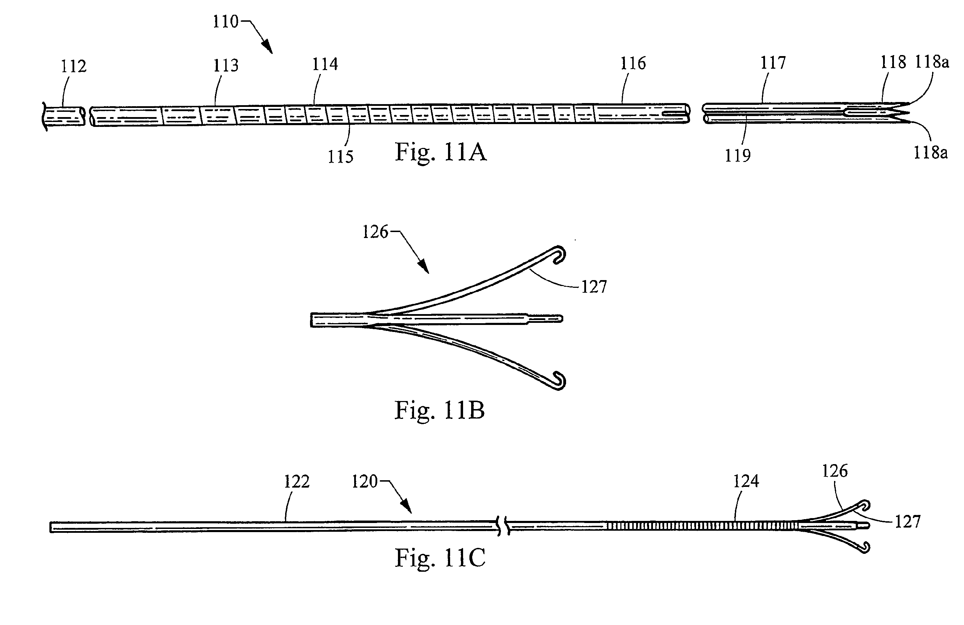

Radiopaque wires may be useful in other, more traditional graspers that are also useful in endoscopic procedures. Figs. 11a-11c depict a three-wire grasper at the distal end of a control rod, and deployed from a flexible cannula. The grasper is deployed via the control rod from the cannula, which also acts as a sheath. The cannula or sheath may be made with a radiopaque material, such as polyimide or fluoropolymer, that has been impregnated or coating with a radiopaque material, such as tungsten, gold, or silver. Alternatively, the sheath may be made from a metal or preferably a radiopaque alloy.

The grasper wires or tongs are preferably made from radiopaque wires, so that the surgeon can then follow the progress of the grasper itself as it emerges from the sheath and is deployed to the area of interest in the patient. The wires are also preferably trained, as described above, so that the wires will assume the grasping position shown in Fig. 11b when they emerge from the sheath. The user grasps the stone or other object by maneuvering the grasper near the stone and then moving the sheath or cannula forward to capture the stone with the wires of the grasper.

Fig. 11a-11c illustrates a cannula 110 having a proximal portion 112, a first distal portion 114 which comprises a spiral cut, an intermediate portion 116 that is not spiral cut, a second distal portion 117, a grasper portion 118, arms 118a, and a central lumen 119. The cannula may comprise a hollow tube of the same material and size discussed above, and the first distal portion 114 may also have material cut in a spiral pattern 115 as shown in Fig. 11a. In one embodiment, the overall length of the cannula is from about four feet to about five feet (about 1.2 m to about 1.5 m), with a preferred length of about fifty-one inches (about 1.3 m). The first distal portion may have material removed in a spiral cut at an angle to a longitudinal axis of the cannula of from about sixty degrees to about eighty degrees. A flexible cannula may have more than one portion having a spiral cut, such as a first spiral-cut portion, an intermediate portion, and a second spiral cut portion. A second intermediate portion may then be interposed between the final spiral-cut portion and a tool or grasper at the end of the cannula.

The cannula with a grasping portion may also comprise a second intermediate portion 113 between the proximal portion 112 of the cannula 110 and the first distal portion 114. The second intermediate portion 113 may comprise from about 0.5 inches (13 mm) to about 2 inches (51 mm) of length of the cannula. The second intermediate portion may be useful in imparting a smaller degree of flexibility to the cannula than the first distal portion 114. The second intermediate portion 113 has a spiral cut also. This spiral cut may be only one-sixth to one-third as long as the first distal portion, and may also have a much larger pitch in its helical cut. Pitch is defined as the axial distance between corresponding points in the helical cut on the outer diameter of the cannula. Thus, in one embodiment, the first distal portion 114 may have a pitch of about 0.021 inches (about 0.5 mm). The second intermediate portion 113 may have a pitch of 0.04 inches (about 1 mm). The pitch of this portion is not limited to a constant value, but may vary as desired to achieve a desired degree of flexibility. In one embodiment, intermediate portion 113 may have an exponentially decreasing pitch, in which the pitch begins at a large value, as much as five times the pitch in the flexible portion 115, and exponentially decreases over several turns, until the pitch reaches the pitch value of the first distal portion. Any pitch may be used that yields a desirable degree of flexibility in this portion of the cannula.

The cannula with a first distal portion and a grasper portion may be used in a grasper for use inside the body of a human being. Other applications may be used for veterinary applications, or other applications in which a flexible grasper may be useful, such as mechanical or hydraulic applications. A flexible cannula 30 with a grasper is depicted in Figs. 11b and 11c. The cannula 120 has a proximal portion 122, a distal portion 124 and a grasper portion 126. In a preferred embodiment, the grasper portion 126 is about 0.1 inches long (about 2.5 mm) and is formed by removing material from the cannula to form three grasper arms. The cannula with grasper may be heat treated or otherwise processed so that when the arms 127 are unrestrained by a sheath or other member, the arms are separated by about 0.40 inches (about 10 mm). A closer view of the grasper portion 126 and arms 127 appears in Fig. 11b. The grasper arms 127 form a continuum with the grasper 126, the distal portion 124 and proximal portion 122.

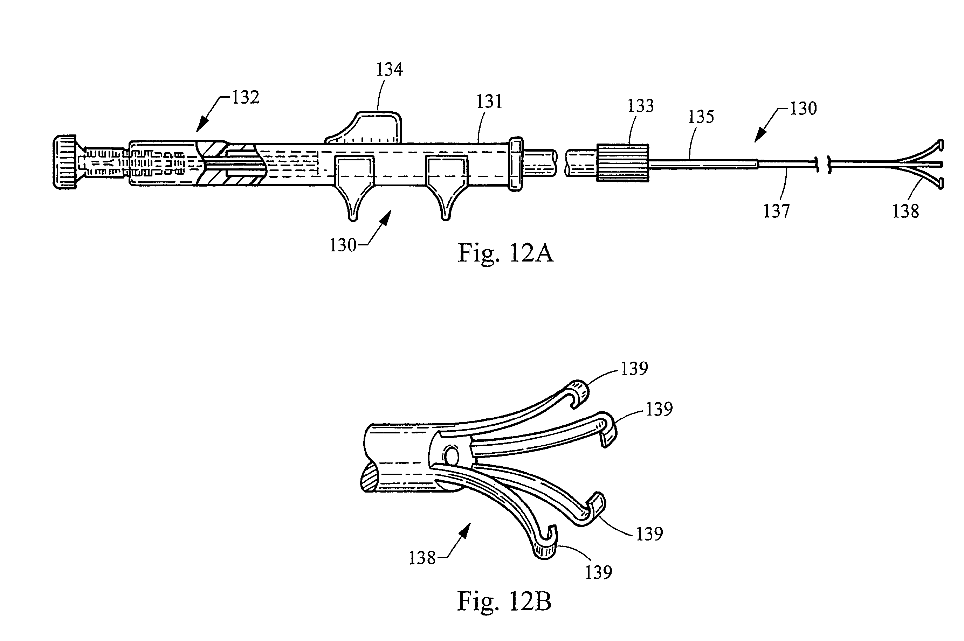

Figs. 12a-12b depict a four-wire grasper with a control handle that performs in a manner similar to a three-wire grasper. The sheath and at least one wire are preferably radiopaque so that the surgeon may more easily maneuver the grasper 130 and manipulate a stone or fragment or other device with a body passage of a patient. The grasper is preferably used by maneuvering the grasper arms near the object to be removed, and the sheath or cannula is then advance via control button 134 on the handle. Alternatively, the grasper portion may be advanced to grasp the stone and then retracted, the arms drawing together as they are retracted into the cannula or sheath.

Grasper 130 may use the flexible cannula 137 in retrieving objects. As shown in Fig. 12a, the grasper comprises a handle 131 with a collet mechanism 132. The control button 134 is connected to flexible cannula 137 for extending or retracting the cannula and grasper portion 138. A sheath 135 that contains the flexible cannula 137 may be connected via sealing connector 133. In operation, the surgeon places the cannula near an object and extends or retracts the cannula 137 to retrieve objects with the grasper 138. Sheath 135 is desirably larger in diameter than the outer diameter of the flexible cannula, so that the cannula can be easily extended from and retracted into the sheath. An end perspective view of the grasper of Fig. 12a is shown in Fig. 12b, depicting the grasper 138 with four arms 139, of which at least one is radiopaque.

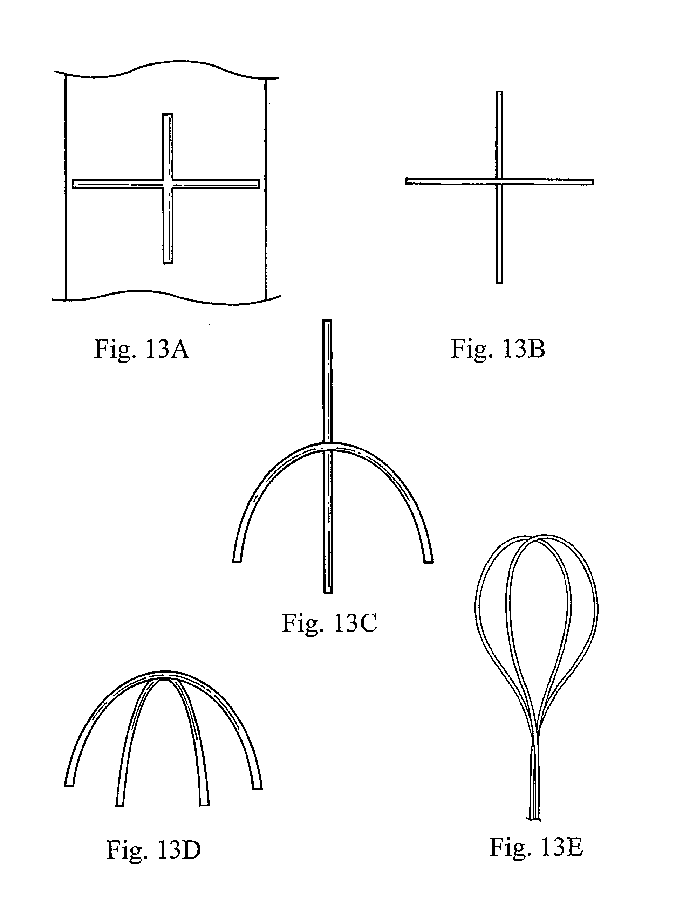

Medical manipulation and retrieval devices may be made from radiopaque wires. In addition, these devices, or at least their end-effectors, such as baskets or grasper arms, may be made from a tube or a sheet of the metallic alloy by using a metal or material removing process. Such processes are illustrated in Figs. 13-14.

Figs. 13a-13e depict a thin strip of radiopaque alloy 13a marked for cutting by a laser or by an electrical discharge machining (EDM) process. The thickness of the strip will correspond to one dimension of the cut material, probably, but not limited to, the thickness dimension. The pattern is programmed into a computer program or computer memory for controlling the cutting. Fig. 13b depicts the material remaining after the cutting process. The "wires" or loops formed by removing material are bent into the desired basket shape in Figs. 13c and 13d. In Fig. 13e, the wires or loops are joined to a cannula or control rod for use in a medical manipulation or retrieval device.

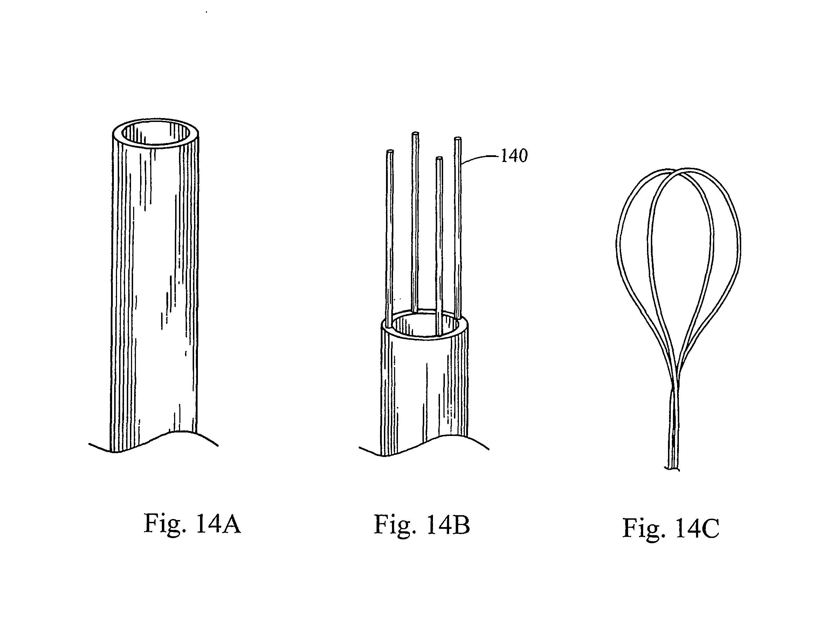

In Figs. 14a-14c, the tubing shown in Fig. 14a is machined to form four components 140 in Fig. 14b. The components may then be joined and shaped to form the basket depicted in Fig. 14c. Alternatively, components 140 may be formed into arms for a grasper. Many end-effectors are possible with these and other metal or material removal processes.

It will be understood that for best visibility, the loops or graspers, or other end-effector of a radiopaque retrieval device are made with a radiopaque alloy. However, radiopacity may be achieved by plating a radiopaque coating, such as a plating of gold or silver, or other radiopaque metal atop another metal, such as stainless steel or a Nitinol superelastic alloy. These embodiments are meant to be included within the scope of the invention. While many superelastic alloys are good candidates for alloying that will impart radiopacity while preserving their superelasticity, other alloys may also be used. For instance, alloys of Cu-Zn-A1 and Cu-Al-Ni exhibit superelasticity and radiopacity.

Alloys with radiopacity may be prepared via vacuum induction melting. The components are charged and formed into an ingot. For instance, nickel, titanium, and a third or fourth element, as described above, may be vacuum melted in an induction furnace and formed into an ingot. The ingot may then be melted under vacuum a second time to ensure consistency throughout the mix. After the alloy is formed, it may be processed as desired to produce wire, tube, sheet, strip and barstock. Wire and tubing are prepared by drawing, the shape of the die determining the final shape, e.g., round wire, pie-shaped wire, or tubing of desired inner and outer diameter. Rectangular wire may also be drawn, and is particularly useful for the arms of grasper embodiments.

It will be recognized that other alloys may also be useful for medical retrieval devices as described herein, alloys which are radiopaque. For example, ASTM F562 alloy, 35-Co 35-Ni, 20-Cr and 10-Mo is radiopaque, and may be used for basket and grasper embodiments. Cobalt-tungsten alloy L605 is also known to be highly radiopaque, as are related alloys UNS R30605, AMS 5537, AMS 5759G, and AMS 5796B. These latter are cobalt based alloys, with about 10 Ni, 20 Cr, and 14-15 percent tungsten.

It is therefore intended that the foregoing detailed description be regarded as illustrative rather than limiting, and that it be understood that it is the following claims, including all equivalents, that are intended to define the scope of this invention.

## Claims

1. A medical manipulation device, comprising:
a control rod (83, 92, 102, 110, 122, 137);
a retrieval device (85, 91, 108, 118, 126, 138) attached to the control rod, the retrieval device comprising a plurality of retrieving elements (87, 108a, 118a, 127, 139), the retrieving elements made from at least one radiopaque wire (31b, 34b) and a plurality of shape memory wires (31, 32); and
a sheath (81, 94, 105, 92, 135) configured so that when the sheath is retracted or the retrieval device is extended, the retrieval device extends from the sheath,
**characterised in that** the at least one radiopaque wire comprises a superelastic radiopaque alloy with from about 3 to about 14 percent opacifying element, and either about 50 percent titanium and the balance nickel, or with about 50 percent nickel and the balance titanium.

2. The medical manipulation device of Claim 1, wherein the opacifying element is selected from the group consisting of silver, tantalum, iron, palladium, platinum, gold, copper, iridium, ruthenium, rhodium, copper, rhenium, tungsten, cobalt, vanadium, chromium, zirconium, niobium, molybdenum, and hafnium.

3. The medical manipulation device of Claim 1, wherein the radiopaque alloy comprises about 50 percent titanium, about 3 to about 14 percent opacifying element, and the balance nickel, wherein the opacifying element is selected from the group consisting of platinum, palladium, silver, copper, gold, iron, rhodium, iridium, ruthenium, and osmium.

4. The medical manipulation device of Claim 1, wherein the radiopaque alloy comprises about 50 percent nickel, about 3 to 14 percent opacifying element, and the balance titanium, wherein the opacifying element is selected from the group consisting of tantalum, tungsten, niobium, molybdenum, zirconium, vanadium, chromium, rhenium, and hafnium.

5. The medical manipulation device according to any of Claims 1-3, wherein the radiopaque alloy comprises about 40-43 atomic percent nickel, about 7 to 10 percent palladium, and the balance titanium.

6. The medical manipulation device according to Claim 5, wherein the radiopaque alloy has a A_{f} from about -20C to about +20C.

7. The medical manipulation device according to any of Claims 1-6, wherein the retrieving elements are selected from the group consisting of loops (87, 91a) of a basket (85, 91), arms (127, 139) of a grasper (126, 138), and coils (108a) of a catcher (108).

8. The medical device according to any of Claims 1-7, wherein the at least one radiopaque wire (32) comprises a Nitinol alloy (32a) with a radiopaque plating (32b).

9. A manipulation device according to any of Claims 1-8, wherein:
the basket (85, 91) is formed with an atraumatic periphery with at least one radiopaque wire loop (31b, 34b) and a plurality of superelastic wire loops (91a) attached to the control rod; and
the sheath (81, 92), configured so that when the sheath is retracted or the basket is extended, the basket expands, and the loops are in a relaxed condition when outside the sheath.

10. The manipulation device of Claim 9, wherein the periphery further comprises a flex point (89a) comprising a bend in a loop or a link for two twisted loops.

11. The manipulation device of Claims 9 and 10, wherein the wire loops form a basket by a first loop (76) wound around a second loop (76) with a third loop (75), the third loop smaller than the first and second loops.

12. The manipulation device of any of Claims 1-11, further comprising a filter mesh (46) connected to the retrieval device.

13. The manipulation device of any of Claims 1-12, further comprising a removable handle (93, 131).

14. A method of making a radiopaque retrieval device according to any of Claims 1-11, wherein material for the radiopaque wire is made via vacuum induction melting of a superelastic alloy, preferably Nitinol alloy with at least one additional metal for imparting radiopacity to the alloy.

15. A method of making a radiopaque retrieval device according to Claim 14 wherein the additional metal selected from the group consisting of tungsten, tantalum, palladium, platinum, gold, iron, iridium, rhenium, rhodium, silver, osmium, ruthenium, cobalt, vanadium, copper, chromium, zirconium, niobium, molybdenum, and hafnium.

## Patentansprüche

1. Medizinisches Manipulationsgerät, umfassend: eine Kontrollstange (83, 92, 102, 110, 122, 137);
eine Rückholvorrichtung (85, 91, 108, 118, 126, 138), die an der Kontrollstange befestigt ist, wobei die Rückholvorrichtung eine Vielzahl von Rückholelementen (87, 108a, 118a, 127, 139) umfasst, wobei die Rückholelemente aus mindestens einem röntgenopaken Draht (31b, 34b) und einer Vielzahl von Formgedächtnisdrähten (31, 32) bestehen; und
eine Schleuse (81, 94, 105, 92, 135), die so konfiguriert ist, dass die Rückholvorrichtung bei Zurückziehen der Schleuse oder Ausfahren der Rückholvorrichtung aus der Schleuse ausgefahren wird, **dadurch gekennzeichnet, dass** der mindestens eine röntgenopake Draht eine superelastische röntgenopake Legierung mit ca. 3 bis ca. 14 Prozent Opazität verleihendem Element und entweder ca. 50 Prozent Titan und Nickel als Rest oder ca. 50 Prozent Nickel und Titan als Rest umfasst.

2. Medizinisches Manipulationsgerät nach Anspruch 1, worin das Opazität verleihende Element aus der Silber, Tantal, Eisen, Palladium, Platin, Gold, Kupfer, Iridium, Ruthenium, Rhodium, Kupfer, Rhenium, Wolfram, Kobalt, Vanadium, Chrom, Zirkonium, Niobium, Molybdän und Hafnium umfassenden Gruppe ausgewählt ist.

3. Medizinisches Manipulationsgerät nach Anspruch 1, worin die röntgenopake Legierung ca. 50 Prozent Titan, ca. 3 bis ca. 14 Prozent Opazität verleihendes Element und Nickel als Rest umfasst, wobei das Opazität verleihende Element aus der Platin, Palladium, Silber, Kupfer, Gold, Eisen, Rhodium, Iridium, Ruthenium und Osmium umfassenden Gruppe ausgewählt ist.

4. Medizinisches Manipulationsgerät nach Anspruch 1, worin die röntgenopake Legierung ca. 50 Prozent Nickel, ca. 3 bis 14 Prozent Opazität verleihendes Element und Titan als Rest umfasst, wobei das Opazität verleihende Element aus der Tantal, Wolfram, Niobium, Molybdän, Zirkonium, Vanadium, Chrom, Rhenium und Hafnium umfassenden Gruppe ausgewählt ist.

5. Medizinisches Manipulationsgerät nach einem der Ansprüche 1-3, worin die röntgenopake Legierung ca. 40-43 Atomprozent Nickel, ca. 7 bis 10 Prozent Palladium und Titan als Rest umfasst.

6. Medizinisches Manipulationsgerät nach Anspruch 5, worin die röntgenopake Legierung einen A_{f} von ca. - 20C bis ca. +20C aufweist.

7. Medizinisches Manipulationsgerät nach einem der Ansprüche 1-6, worin die Rückholelemente aus der Schlaufen (87, 91a) eines Korbs (85, 91), Arme (127, 139) eines Greifers (126, 138) und Spulen (108a) einer Fangvorrichtung (108) umfassenden Gruppe ausgewählt sind.

8. Medizinisches Manipulationsgerät nach einem der Ansprüche 1-7, worin der mindestens eine röntgenopake Draht (32) eine Nitinol-Legierung (32a) mit einer röntgenopaken Auflage (32b) umfasst.

9. Medizinisches Manipulationsgerät nach einem der Ansprüche 1-8, worin: der Korb (85, 91) mit einer atraumatischen Peripherie mit mindestens einer röntgenopaken Drahtschlaufe (31b, 34b) und einer Vielzahl von superelastischen Drahtschlaufen (91a), die an der Kontrollstange befestigt sind, geformt ist; und die Schleuse (81, 92) so konfiguriert ist, dass sich der Korb aufweitet, wenn die Schleuse zurückgezogen wird oder der Korb ausgefahren wird, und die Schlaufen sich außerhalb der Schleuse in einem entspannten Zustand befinden.

10. Manipulationsgerät nach Anspruch 9, worin die Peripherie ferner einen Biegungspunkt (89a) mit einer Biegung in einer Schlaufe oder einem Verbindungsglied für zwei verdrehte Schlaufen umfasst.

11. Manipulationsgerät nach Anspruch 9 und 10, worin die Drahtschlaufen einen Korb bilden, indem eine erste Schlaufe (76) um eine zweite Schlaufe (76) mit einer dritten Schlaufe (75) gewickelt ist, wobei die dritte Schlaufe kleiner ist als die erste und zweite Schlaufe.

12. Manipulationsgerät nach einem der Ansprüche 1-11, das ferner ein mit der Rückholvorrichtung verbundenes Maschenfilter (46) umfasst.

13. Manipulationsgerät nach einem der Ansprüche 1-12, das ferner einen entfernbaren Handgriff (93, 131) umfasst.

14. Verfahren zur Herstellung einer röntgenopaken Rückholvorrichtung nach einem der Ansprüche 1-11, worin das Material für den röntgenopaken Draht durch Vakuuminduktionsschmelzen einer superelastischen Legierung, vorzugsweise einer Nitinol-Legierung mit mindestens einem zusätzlichen Metall, um der Legierung Röntgenopazität zu verleihen, hergestellt wird.

15. Verfahren zur Herstellung einer röntgenopaken Rückholvorrichtung nach Anspruch 14, worin das zusätzliche Metall aus der Wolfram, Tantal, Palladium, Platin, Gold, Eisen, Iridium, Rhenium, Rhodium, Silber, Osmium, Ruthenium, Kobalt, Vanadium, Kupfer, Chrom, Zirkonium, Niobium, Molybdän und Hafnium umfassenden Gruppe ausgewählt ist.

## Revendications

1. Dispositif médical de manipulation comprenant : une tige de commande (83, 92, 102, 110, 122, 137),
un dispositif de récupération (85, 91, 108, 118, 126, 138) relié à la tige de commande, le dispositif de récupération comprenant plusieurs éléments de récupération (87, 108a, 118a, 127, 139), les éléments de récupération étant constitués d'au moins un fil opaque aux rayons X (31b, 34b) et de plusieurs fils à mémoire de forme (31, 32) et
une gaine (81, 94, 105, 92, 135) configurée de manière à ce que le dispositif de récupération déborde de la gaine lorsque l'on rétracte la gaine ou lorsque l'on déploie le dispositif de récupération, **caractérisé en ce que**
le ou les fils opaques aux rayons X sont constitués d'un alliage super élastique opaque aux rayons X constitué d'environ 3 à environ 14 pourcent d'un élément opacifiant et d'environ 50 pourcent de titane, le reste de l'alliage étant constitué de nickel, ou d'environ 50 pourcent de nickel, le reste de l'alliage étant constitué de titane.

2. Dispositif médical de manipulation selon la revendication 1, dans lequel l'élément opacifiant est sélectionné dans l'ensemble constitué de l'argent, du tantale, du fer, du palladium, du platine, de l'or, du cuivre, de l'iridium, du ruthénium, du rhodium, du cuivre, du rhénium, du tungstène, du cobalt, du vanadium, du chrome, du zirconium, du niobium, du molybdène et de l'hafnium.

3. Dispositif médical de manipulation selon la revendication 1, dans lequel l'alliage opaque aux rayons X contient environ 50 pourcent de titane, d'environ 3 à environ 14 pourcent d'éléments opacifiants, le reste de l'alliage étant constitué de nickel, l'élément opacifiant étant sélectionné dans l'ensemble constitué du platine, du palladium, de l'argent, du cuivre, de l'or, du fer, du rhodium, de l'iridium, du ruthénium et de l'osmium.

4. Dispositif médical de manipulation selon la revendication 1, dans lequel l'alliage opaque aux rayons X contient environ 50 pourcent de nickel, d'environ 3 à environ 14 pourcent d'éléments opacifiants, le reste de l'alliage étant constitué du titane, l'élément opacifiant étant sélectionné dans l'ensemble constitué du tantale, du tungstène, du niobium, du molybdène, du zirconium, du vanadium, du chrome, du rhénium et de l'hafnium.

5. Dispositif médical de manipulation selon l'une quelconque des revendications 1 à 3, dans lequel l'alliage opaque aux rayons X contient d'environ 40 à 43 pourcent en atomes de nickel, d'environ 7 à 10 pourcent de palladium, le reste de l'alliage étant constitué de titane.

6. Dispositif médical de manipulation selon la revendication 5, dans lequel l'alliage opaque aux rayons X présente un A_{f} compris entre environ -20C et environ +20C.

7. Dispositif médical de manipulation selon l'une quelconque des revendications 1 à 6, dans lequel les éléments de récupération sont sélectionnés dans l'ensemble constitué des boucles (87, 91a), d'un panier (85, 91), de bras (127, 139), d'une pince (126, 138) et de bobines (108a) d'un dispositif de saisie (108).

8. Dispositif médical selon l'une quelconque des revendications 1 à 7, dans lequel au moins un fil opaque aux rayons X (32) contient un alliage de Nitinol (32a) doté d'un placage opaque aux rayons X (32b).

9. Dispositif de manipulation selon l'une quelconque des revendications 1 à 8, dans lequel :
le panier (85, 91) est constitué d'un périmètre atraumatique doté d'au moins une boucle (31b, 34b) en fil opaque aux rayons X et de plusieurs boucles (91a) en fil super élastique reliées à la tige de commande et
la gaine (81, 92) est configurée de telle sorte que lorsque la gaine se rétracte ou lorsque le panier est déployé, le panier se dilate, les boucles étant à l'état détendu lorsqu'elles sont à l'extérieur de la gaine.

10. Dispositif de manipulation selon la revendication 9, dans lequel le périmètre comprend de plus un point de fléchissement (89a) comprenant un coude dans une boucle ou une liaison de deux boucles torsadées.

11. Dispositif de manipulation selon les revendications 9 et 10, dans lequel les boucles de fil forment un panier par une première boucle (76) enroulée autour d'une deuxième boucle (76) par une troisième boucle (75), la troisième boucle étant plus petite que la première boucle et que la deuxième boucle.

12. Dispositif de manipulation selon l'une quelconque des revendications 1 à 11, comprenant de plus des mailles de filtration (46) reliées au dispositif de récupération.

13. Dispositif de manipulation selon l'une quelconque des revendications 1 à 12, comprenant de plus une poignée amovible (93, 131).

14. Procédé pour rendre opaque aux rayons X un dispositif de récupération selon l'une quelconque des revendications 1 à 11, dans lequel le matériau du fil opaque aux rayons X est constitué par fusion par induction sous vide d'un alliage super élastique, de préférence un alliage de Nitinol avec au moins un métal supplémentaire, ce qui permet de rendre l'alliage opaque aux rayons X.

15. Procédé selon la revendication 14 pour rendre opaque aux rayons X un dispositif de récupération, dans lequel le métal supplémentaire est sélectionné dans l'ensemble constitué du tungstène, du tantale, du palladium, du platine, de l'or, du fer, de l'iridium, du rhénium, du rhodium, de l'argent, de l'osmium, du ruthénium, du cobalt, du vanadium, du cuivre, du chrome, du zirconium, du niobium, du molybdène et de l'hafnium.
